# EUROPEAN PATENT APPLICATION

(11) **EP 4 681 778 A2**
(43) Date of publication of application: **21.01.2026**
(21) Application number: 25221700.5
(22) Date of filing: 02.04.2020
(51) Int. Cl.: A61P 31/12

(54) **COMBINATION THERAPIES BASED ON PD1 AND IL-17B INHIBITORS**

(30) Priority: 03.04.2019 EP 19305438
(62) Divisional of application: 20718247.8
(71) Applicant: Orega Biotech, 69009 Lyon (FR); UNIVERSITE DE MONTPELLIER, 34090 Montpellier (FR); Institut National de la Sante et de la Recherche Medicale (INSERM), 75013 Paris (FR); Institut Régional du Cancer de Montpellier, 34090 Montpellier (FR)
(72) Inventor: BONNEFOY, Nathalie, 34980 SAINT CLEMENT DE RIVIERE (FR); BASTID, Jérémy, 69160 TASSIN LA DEMI LUNE (FR)
(74) Representative: Ipsilon

(57) **Abstract**

The present invention concerns the combination of PD1 and IL-17B inhibitors, especially for the treatment of patients and diseases resistant to anti-PD1 therapies.

## Description

### FIELD OF THE INVENTION

The present invention relates to combination therapies based on PD1 and IL-17B inhibitors.

### BACKGROUND OF THE INVENTION

Immune checkpoints are regulators of the immune system. These pathways are crucial for self-tolerance, which prevents the immune system from attacking cells indiscriminately.

The chronicity of cancer and persistent infections provides constant antigen exposure to antigen-reactive T-cells, leading to cellular exhaustion and abrogation of effector functions. The expression of cell surface-bound molecules such as programmed cell death protein 1 (PD1/PD-1/CD279) and cytotoxic T lymphocyte-associated protein 4 (CTLA4/CTLA-4/CD152) on antigen-specific T-cells are markers of exposure to immunogenic stimuli. PD1 and CTLA4 identify as immune checkpoints due to their crucial role in down-regulating the magnitude of T-cell responses. Other immune checkpoint molecules of clinical relevance are the T-cell immunoglobulin and mucin-domain containing 3 (TIM3/TIM-3), with its nominal ligand galectin 9, and to a lesser extent the lymphocyte-activation gene 3 (LAG3/LAG-3/CD223), which binds to major histocompatibility complex (MHC) class II molecules with higher affinity than the CD4 receptor.

PD1 expression on T-cells is rapidly induced after antigen exposure, following engagement between the T-cell receptor (TCR) and its cognate epitope-loaded MHC molecule in the draining lymph nodes. In addition to TCR-dependence, the presence of interleukin 2 (IL-2), IL-7, IL-15, vascular endothelial growth factor (VEGF), IL-6, and transforming growth factor beta (TGF-β) in the local cytokine milieu in lymph nodes and diseased tissue additionally contribute to PD1 up-regulation on T-cells. PD1 interacts with its ligands, namely PDL-1 (PDL1/B7-H1/CD274) and PDL-2 (PDL2/B7-DC/CD273). The PD1 ligands are expressed on transformed cells, professional antigen-presenting cells (pAPCs), and epithelial cells, as well as T-cells. This interaction provides signals that tolerize T-cells to their antigenic targets, disarming their effector functions. Type 1 interferons (IFN-α/β) and tumor necrosis factor alpha (TNF-α) can up-regulate PDL1 expression on T-cells, B-cells, natural killer (NK) cells, myeloid cells, and epithelial cells, while PDL2 expression is inducible via interferon gamma (IFN-γ), granulocyte-macrophage colony-stimulating factor (GM-CSF), and IL-4 signaling.

By targeting factors that foster the development and maintenance of an immunosuppressive microenvironment, e.g. within tumors, immunotherapies can release the brakes on the host's own immune system and possibly cure of disease.

In practice, checkpoint inhibitors (CPi) targeting PD1 or its ligand PDL1 have demonstrated unprecedented clinical efficacy in more than 15 cancer types including melanoma, non-small cell lung cancer (NSCLC), renal cell carcinoma (RCC), bladder carcinoma and Hodgkin's lymphoma. Indeed, therapeutic mAbs targeting either PD1 or PDL1 block ligand/receptor interactions to release T-cells from their exhausted phenotype and allow for reinvigoration of tumor antigen-specific immunity (for a review, see O'Donnell et al., Cancer treatment Reviews 52 (2017), 71-81).

As the phenomenon of T-cell exhaustion in chronic infections is similar to what observed in cancer, the same strategy has been applied to chronic infectious diseases such as tuberculosis (TB), human immunodeficiency virus (HIV), hepatitis and malaria (for a review, see Rao et al., International Journal of Infectious Diseases 56 (2017), 221-228).

Nevertheless, primary resistance to anti-PD1 therapies is common, affecting up to 60% of patients in some cancer types. Furthermore, it is now becoming apparent that encouraging initial responses observed amongst some patients can be undone by their development of acquired resistance to anti-PD1 therapies (referred to throughout as acquired resistance) leading to disease relapse. A substantial effort is currently underway to fully elucidate the mechanisms by which anti-PD1/PDL1 therapies exert their efficacy, to understand mechanisms of resistance present within some patients that limit their activity, and to develop *a priori* combination therapeutic approaches to sensitize resistant patients. In practice and as reviewed in O'Donnell et al. (Cancer treatment Reviews 52 (2017), 71-81), different combination strategies have been proposed or developed to augment the therapeutic activity of anti-PD1/PDL1 antibodies, including but not limited to: anti-CTLA4 therapy, anti-OX40 (CD134) therapy, anti-TIM3, anti-LAG3 and anti-TIGIT therapies, radiotherapy, chemo-therapies such as doxorubicin, BRAF/MEK inhibitors, cytokine therapy such as anti-VEGF mAbs (bevacizumab), anti-CD40, anti-CD73 or anti-CD137 antibodies, viral therapies using oncolytic viruses, ...

However, there is still a need to develop more efficient strategies for treating said kind of diseases.

### SUMMARY OF THE INVENTION

The present invention relates to combination therapies for the treatment of e.g. cancer or infectious diseases. In particular, the present invention is defined by the claims.

### DETAILED DESCRIPTION OF THE INVENTION

Programmed cell death protein 1 (PD1) is an efficient therapeutic target for various types of cancer. However, some tumors are refractory to anti-PD1. In the present application, the inventors reveal the link between the PD1 immune checkpoint and IL-17B. They found that the simultaneous inhibition of PD1 and IL-17B is efficient versus different types of cancer. Collectively, the data reveal the first evidence indicating the importance of IL-17B for resistance against anti-PD1 therapeutics and suggest blocking IL-17B in combination with anti-PD1 as a novel therapeutic strategy to combat diseases involving the PD1 immune checkpoint.

Accordingly, the first object of the present invention relates to a composition comprising a PD1 inhibitor and an IL-17B inhibitor.

According to a specific embodiment, a composition according to the invention herein has an amount of the PD1 inhibitor inferior to its amount in a composition not comprising an IL-17B inhibitor.

According to another aspect, the invention relates to the use of such a composition in therapy, advantageously for the treatment of a cancer or an infectious disease as detailed below.

According to a second aspect and advantageously in relation to the treatment of a cancer or an infectious disease, the invention concerns a composition comprising an IL-17B inhibitor for use in administering a subject treated with a PD1 inhibitor. According to a specific embodiment, the subject is resistant to the treatment with the PD1 inhibitor. In other words, the invention also relates to a composition comprising an IL-17B inhibitor for use in increasing the sensitivity of a subject to a PD1 inhibitor.

As used therein, the expression "resistant to PD1 inhibitors" can refer to the fact that:
- The majority of the patients having a given disease do not respond to these treatments (PD1 inhibitors) and/or have a poor prognostic. In that case, the disease is considered to be globally resistant to anti-PD1 treatments, whereas other diseases are sensitive to said treatments. As an example, certain types of cancer are known to be more resistant than others to PD1 inhibitors; and/or
- Even if a disease is globally known to be sensitive to anti-PD1 treatments, a given patient having said disease can be resistant to anti-PD1 therapies. This wording covers primary resistance to said therapies as well as acquired resistance to said therapies as defined above. According to another embodiment, a patient being resistant can be a patient with hyperprogressive disease (HPD) following anti PD1 treatment, i.e. with accelerated disease upon treatment with PD1 inhibitors.

A further aspect of the invention concerns a method for enhancing the potency of a PD1 inhibitor administered to a patient as part of a treatment regimen, the method comprising administering to the subject a pharmaceutically effective amount of an IL-17B inhibitor in combination with the PD1 inhibitor. In other words, the invention concerns a composition comprising an IL-17B inhibitor for use in increasing the efficacy of a treatment with a PD1 inhibitor in a subject. It further concerns a method of treating a patient in need thereof comprising administering to the patient a therapeutically effective combination of a PD1 inhibitor with an IL-17B inhibitor, wherein administration of the combination results in enhanced therapeutic efficacy relative to the administration of the PD1 inhibitor alone.

As used herein, the expression "enhancing the potency or efficacy of a PD1 inhibitor" refers to the ability of the IL-17B inhibitor to increase the ability of the PD1 inhibitor to inhibit the progression of the disease and then to improve the therapeutic outcome.

In the frame of the invention, the treatment is advantageously dedicated to a disease involving the PD1 immune checkpoint. As previously reported in the literature, said disease is advantageously selected in the group consisting of: cancer and infectious diseases, especially chronic infections. An infectious disease is advantageously selected in the following group: severe sepsis, septic shock, viral infections especially infections by human immunodeficiency virus (HIV), hepatitis virus, particularly hepatitis B virus (HBV) and hepatitis C virus (HCV), cytomegalovirus or Epstein-Barr virus, fungal infections such as mucormycosis, mosquito-borne infectious diseases such as malaria, and bacterial infections such as tuberculosis (TB).

As used herein and as illustrated in relation to cancer, the expression "enhanced therapeutic efficacy" refers to a slowing or diminution of the growth of cancer cells or a solid tumor, or a reduction in the total number of cancer cells or total tumor burden. An "improved therapeutic outcome" or "enhanced therapeutic efficacy" therefore means there is an improvement in the condition of the patient according to any clinically acceptable criteria, including, for example, decreased tumor size, a delayed tumor progression, increased progression-free survival, increased overall survival time, an increase in life expectancy, or an improvement in quality of life. In particular, "improved" or "enhanced" refers to an improvement or enhancement of 1%, 5%, 10%, 25% 50%, 75%, 100%, or greater than 100% of any clinically acceptable indicator of therapeutic outcome or efficacy. As used herein, the expression "relative to" when used in the context of comparing the activity and/or efficacy of a combination composition comprising the PD1 inhibitor with the IL-17B inhibitor to the activity and/or efficacy of the PD1 inhibitor alone, refers to a comparison using amounts known to be comparable according to one of skill in the art.

As used herein, the term "cancer" has its general meaning in the art and includes, but is not limited to, solid tumors and blood-borne tumors. The term cancer includes diseases of the skin, tissues, organs, bone, cartilage, blood and vessels. The term "cancer" further encompasses both primary and metastatic cancers. Examples of cancers that may be treated by methods and compositions of the invention include, but are not limited to, cancer cells from the bladder, blood, bone, bone marrow, brain, breast, colon, oesophagus, gastrointestinal tract, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; undifferentiated carcinoma; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; Paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; Leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; Ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

In some embodiments, the methods and compositions of the present invention are particularly suitable for the treatment of the following cancers: melanoma, sarcoma, Cutaneous Squamous Cell Carcinoma (CSCC), Primary Mediastinal Large B-Cell Lymphoma (PMBCL), Microsatellite Instability-High Cancer (MSI-H), Hepatocellular Carcinoma (HCC), Small Cell Lung Cancer, Non-Small Cell Lung Cancer (NSCLC), ovarian cancer, Merkel Cell Carcinoma (MCC), Head and Neck Squamous Cell Carcinoma (HNSCC), Cervical Cancer, gastric cancer, esophageal cancer, urothelial carcinoma, Renal Cell Carcinoma (RCC), bladder carcinoma, anal cancer, triple negative breast cancer (TNBC), mesothelioma and Hodgkin's lymphoma (cHL).

In some embodiments, methods and compositions of the present invention are particularly suitable for the treatment of diseases, especially cancers, resistant to PD1 inhibitors. As used herein, the term "resistant" refers to the repeated outbreak of the disease, or a progression of the disease independently of whether the disease was cured before said outbreak or progression.

"Antineoplastic resistance" is the drug resistance of neoplastic (cancerous) cells, or the ability of cancer cells to survive and grow despite anti-cancer therapies. There are two general causes of antineoplastic therapy failure: Inherent properties, such as genetic characteristics, giving cancer cells their resistance, which is rooted in the concept of cancer cell heterogeneity and acquired resistance after drug exposure. Cancer cells can become resistant to drugs by various mechanisms, including: altered membrane transport, enhanced DNA repair, apoptotic pathway defects, alteration of target molecules, protein and pathway mechanisms, such as enzymatic deactivation. Since cancer is a genetic disease, two genomic events underlie these mechanisms of acquired drug resistance: Genome alterations (e.g. gene amplification and deletion) and epigenetic modifications (Housman et al., Cancer, 2014, 6, 1769-1792).

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

As used herein the "PD1" has its general meaning in the art and refers to as a programmed cell death protein 1 (PD1/PD-1/CD279). As used herein, the terms "programmed cell death protein 1", "PD1", "PD-1", and "CD279" are used interchangeably. By "PD1 ligand" is meant a polypeptide which binds to and/or activates PD1, especially PDL1 and PDL2.

As used herein the term "PD1 inhibitor" refers to an agent which interferes with PD1 activation or function. Examples of PD1 inhibitors include PD1 antibodies (e.g. PD1, PDL1 or PDL2 antibodies); small organic molecule PD1 antagonists; and/or agents that bind to, or interfere with function of PD1. Typically, the PD1 inhibitor is an antibody or small organic molecule which binds to the PD1 receptor or to its ligand PDL1 or PDL2.

In some embodiments, the PD1 inhibitor is a small organic molecule. As used herein, the term "small organic molecule" refers to a molecule of size comparable to those organic molecules generally used in pharmaceuticals. The term excludes biological macromolecules (e.g.; proteins, nucleic acids, etc.); preferred small organic molecules range in size up to 2000 Da, and most preferably up to about 1000 Da.

Patent publications related to PD1/PDL1 antibodies include: WO2018/204303, CN108640992, WO2018/036472, CN107384933, WO2015/035606, CN107043425, CN106939050, CN106749663.

A non-exhaustive list of PD1/PDL1 antibodies comprises: Pembrolizumab (Keytruda^{®}), Nivolumab (Opdivo^{®}), BMS-936559 (MDX 1105), Cemiplimab (REGN2810), Cemiplimab-rwlc (LIBTAYO^{®}), Avélumab (MSB0010718C or Bavencio), Durvalumab (MEDI4736 or INFIMZI^{®}), Atezolizumab (MPDL3280A or Tecentriq^{®}), Spartalizumab (PDR 001), as well as their combination.

According to specific embodiments:
- Atezolizumab or Avélumab or Durvalumab or Pembrolizumab or Nivolumab can be used for treating bladder cancer;
- Pembrolizumab or Nivolumab can be used for treating colorectal cancer or gastric cancer or head and neck cancer or Hodgkin's lymphoma;
- Nivolumab can be used for treating hepatocellular cancer;
- Nivolumab or Pembrolizumab or Ipilimumab and Nivolumab can be used for treating melanoma;
- Atezolizumab or Durvalumab or Pembrolizumab or Nivolumab can be used for treating lung cancer;
- Avélumab can be used for treating Merkel cell cancer;
- Pembrolizumab can be used for treating tissue-agnostic cancer.

The interleukin 17 (IL-17) family comprises 6 interleukins (IL-17A, IL-17B, IL- 17C, IL-17D, IL-17E and IL-17F) and their receptors (IL-17RA, IL-17RB, IL- 17RC, IL-17RD and IL-17RE) (Gaffen, S. L. (2009) Nature reviews. Immunology 9(8): 556-567). IL-17B binds the dimeric IL-17RB receptor and IL-17E binds a complex of IL-17RA and IL-17RB.

As used herein the term "IL-17B" has its general meaning in the art and a polypeptide having a sequence according to GenBank Acc. No. NP_001304916.1 or NP_055258.1, the product of the human IL-17B gene, and include all of the variants, isoforms or species homologs of IL-17B. As used herein, the term "IL-17B signaling" as used herein means the processes initiated by IL-17B or a second IL-17B receptor ligand interacting with the IL-17RB receptor on the cell surface, resulting in measurable changes in cell function. Typically, IL-17B signaling can be assessed by functional assays measuring for example effect of IL-17B receptor ligand on cell proliferation or differentiation, or using reporter genes and reporter gene constructs.

As used herein, the term "IL17RB" (IL-17RB, CRL4, EVI27, IL17RH1, or MGC5245) as used herein means "interleukin 17 receptor B", a polypeptide having an amino acid sequence according to GenBank Acc. No. NP061195, the product of the human IL17RB receptor gene, and include all of the variants, isoforms and species homologs of IL17RB.

Accordingly, as used herein the terms "IL-17B inhibitors" refers to any compound that is able to inhibit the IL-17B signaling. The IL-17B inhibitor to be used in the methods and compositions described herein is a molecule that blocks, suppresses, or reduces (including significantly) the biological activity of the IL-17B cytokine, including downstream pathways mediated by IL-17B signaling. Thus the term "IL-17B inhibitor" implies no specific mechanism of biological action whatsoever, and is deemed to expressly include and encompass all possible pharmacological, physiological, and biochemical interactions with IL-17B whether direct or indirect.

In some embodiments, the IL-17B inhibitor is selected from the group consisting of antibodies directed against IL-17B and antibodies directed against a receptor of IL-17B (e.g., an antibody specifically binds IL-17RB or the dimeric complex formed thereby). According to a specific embodiment, the IL-17RB inhibitor is an IL-17E inhibitor, e.g. an antibody that specifically binds IL-17E.

Patent publications related to IL17B/IL17RB antibodies include: WO2010/116123, WO2011/044563, WO2016/004045, US2009/0291097.

IL17B/IL17RB antibodies are commercially available, e.g. the mouse IL-17B antibody (AF1709 ; R&D Systems) or the human IL-17RB antibody (MAB1207 ; R&D Systems).

As used herein, the term "antibody" is thus used to refer to any antibody-like molecule that has an antigen binding region, and this term includes antibody fragments that comprise an antigen binding domain such as Fab', Fab, F(ab')2, single domain antibodies (DABs), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lamda) bodies (scFv-CL fusions); BiTE (Bispecific T-cell Engager, scFv-scFv tandems to attract T cells); DVD-Ig (dual variable domain antibody, bispecific format); SIP (small immunoprotein, a kind of minibody); SMIP ("small modular immunopharmaceutical" scFv-Fc dimer; DART (ds-stabilized diabody "Dual Affinity ReTargeting"); small antibody mimetics comprising one or more CDRs and the like. The techniques for preparing and using various antibody-based constructs and fragments are well known in the art (see Kabat et al., 1991, specifically incorporated herein by reference). Diabodies, in particular, are further described in EP 404, 097 and WO 93/1 1 161; whereas linear antibodies are further described in Zapata et al. (1995). Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well known and described in the art. For example, each of Beckman et al., 2006; Holliger & Hudson, 2005; Le Gall et al., 2004; Reff & Heard, 2001; Reiter et al., 1996; and Young et al., 1995 further describe and enable the production of effective antibody fragments. In some embodiments, the antibody of the present invention is a single chain antibody. As used herein the term "single domain antibody" has its general meaning in the art and refers to the single heavy chain variable domain of antibodies of the type that can be found in Camelid mammals which are naturally devoid of light chains. Such single domain antibody are also "nanobody^{®}". For a general description of (single) domain antibodies, reference is also made to the prior art cited above, as well as to EP 0 368 684, Ward et al. (Nature 1989 Oct 12; 341 (6242): 544-6), Holt et al., Trends Biotechnol., 2003, 21(11):484-490; and WO 06/030220, WO 06/003388.

In some embodiments, the antibody is a humanized antibody. As used herein, "humanized" describes antibodies wherein some, most or all of the amino acids outside the CDR regions are replaced with corresponding amino acids derived from human immunoglobulin molecules. Methods of humanization include, but are not limited to, those described in U.S. Pat. Nos. 4,816,567, 5,225,539, 5,585,089, 5,693,761, 5,693,762 and 5,859,205, which are hereby incorporated by reference.

In some embodiments, the antibody is a fully human antibody. Fully human monoclonal antibodies also can be prepared by immunizing mice transgenic for large portions of human immunoglobulin heavy and light chain loci. See, e.g., U.S. Pat. Nos. 5,591,669, 5,598,369, 5,545,806, 5,545,807, 6,150,584, and references cited therein, the contents of which are incorporated herein by reference. These animals have been genetically modified such that there is a functional deletion in the production of endogenous (e.g., murine) antibodies. The animals are further modified to contain all or a portion of the human germ-line immunoglobulin gene locus such that immunization of these animals will result in the production of fully human antibodies to the antigen of interest. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (KAMA) responses when administered to humans. In vitro methods also exist for producing human antibodies. These include phage display technology (U.S. Pat. Nos. 5,565,332 and 5,573,905) and in vitro stimulation of human B cells (U.S. Pat. Nos. 5,229,275 and 5,567,610). The contents of these patents are incorporated herein by reference.

In some embodiments, the antibody does not comprise an Fc portion that induces antibody dependent cellular cytotoxicity (ADCC). The terms "Fc domain," "Fc portion," and "Fc region" refer to a C-terminal fragment of an antibody heavy chain, e.g., from about amino acid (aa) 230 to about aa 450 of human gamma heavy chain or its counterpart sequence in other types of antibody heavy chains (e.g., α, δ, ε and µ for human antibodies), or a naturally occurring allotype thereof. Unless otherwise specified, the commonly accepted Kabat amino acid numbering for immunoglobulins is used throughout this disclosure (see Kabat et al. (1991 ) Sequences of Protein of Immunological Interest, 5th ed., United States Public Health Service, National Institute of Health, Bethesda, MD). In some embodiments, the antibody of the present invention does not comprise an Fc domain capable of substantially binding to a FcgRIIIA (CD16) polypeptide. In some embodiments, the antibody of the present invention lacks an Fc domain (e.g. lacks a CH2 and/or CH3 domain) or comprises an Fc domain of IgG2 or IgG4 isotype. In some embodiments, the antibody of the present invention consists of or comprises a Fab, Fab', Fab'-SH, F (ab') 2, Fv, a diabody, single-chain antibody fragment, or a multispecific antibody comprising multiple different antibody fragments. In some embodiments, the antibody of the present invention is not linked to a toxic moiety. In some embodiments, one or more amino acids selected from amino acid residues can be replaced with a different amino acid residue such that the antibody has altered C2q binding and/or reduced or abolished complement dependent cytotoxicity (CDC). This approach is described in further detail in U.S. Patent Nos. 6,194,551.

According to a further embodiment, the inhibitory activity against PD1 and IL-17B is harboured by a single molecule, i.e. a bispecific inhibitor. According to a specific embodiment, such an inhibitor is a bispecific antibody which comprises a first antigen-binding domain that binds to PD1 and a second antigen-binding domain that binds to IL-17B or IL-17RB or IL-17E.

In some embodiments, the PD1 or IL-17B inhibitor is an inhibitor of PD1, PDL1, PDL2, IL-17B or IL-17RB expression. An "inhibitor of expression" refers to a natural or synthetic compound that has a biological effect to inhibit the expression of a gene. In a preferred embodiment of the invention, said inhibitor of gene expression is a siRNA, an antisense oligonucleotide or a ribozyme. For example, anti-sense oligonucleotides, including anti-sense RNA molecules and anti-sense DNA molecules, would act to directly block the translation of PD1 or IL-17B mRNA by binding thereto and thus preventing protein translation or increasing mRNA degradation, thus decreasing the level of PD1or IL-17B, and thus activity, in a cell. For example, antisense oligonucleotides of at least about 15 bases and complementary to unique regions of the mRNA transcript sequence encoding PD1 or IL-17B can be synthesized, e.g., by conventional phosphodiester techniques. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732). Small inhibitory RNAs (siRNAs) can also function as inhibitors of expression for use in the present invention. PD1 or IL-17B gene expression can be reduced by contacting a patient or cell with a small double stranded RNA (dsRNA), or a vector or construct causing the production of a small double stranded RNA, such that PD1 or IL-17B gene expression is specifically inhibited (i.e. RNA interference or RNAi). Antisense oligonucleotides, siRNAs, shRNAs and ribozymes of the invention may be delivered in vivo alone or in association with a vector. In its broadest sense, a "vector" is any vehicle capable of facilitating the transfer of the antisense oligonucleotide, siRNA, shRNA or ribozyme nucleic acid to the cells and typically cells expressing PD1 or IL-17B. Typically, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide, siRNA, shRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

As used herein the term "co-administering" as used herein means a process whereby the combination of the IL-17B inhibitor and the PD1 inhibitor is administered to the same patient. The IL-17B inhibitor and the PD1 inhibitor may be administered simultaneously, at essentially the same time, or sequentially. The IL-17B inhibitor and the PD1 inhibitor need not be administered by means of the same vehicle. The IL-17B inhibitor and the PD1 inhibitor may be administered one or more times and the number of administrations of each component of the combination may be the same or different. In addition, the IL-17B inhibitor and the PD1 inhibitor need not to be administered at the same site.

As used herein, the term "therapeutically effective combination" as used herein refers to an amount or dose of an IL-17B inhibitor together with the amount or dose of the PD1 inhibitor that is sufficient to treat the disease, especially cancer. The amount of the IL-17B inhibitor in a given therapeutically effective combination may be different for different individuals and different tumor types, and will be dependent upon the one or more additional agents or treatments included in the combination. The "therapeutically effective amount" is determined using procedures routinely employed by those of skill in the art such that an "improved therapeutic outcome" results. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

According to the invention, the IL-17B inhibitor and the PD1 inhibitor are administered to the patient in the form of a pharmaceutical composition. Typically, the IL-17B inhibitor and the PD1 inhibitor may be combined with pharmaceutically acceptable excipients, and optionally sustained-release matrices, such as biodegradable polymers, to form therapeutic compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The IL-17B inhibitor and the PD1 inhibitor can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the typical methods of preparation are vacuum-drying and freeze-drying techniques which yield a powder of the active ingredient plus any additional desired ingredient from a previously sterile-filtered solution thereof. The preparation of more, or highly concentrated solutions for direct injection is also contemplated, where the use of DMSO as solvent is envisioned to result in extremely rapid penetration, delivering high concentrations of the active agents to a small tumor area. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the patient being treated. The person responsible for administration will, in any event, determine the appropriate dose for the individual patient.

According to other embodiments, the administration of the PD1 and IL-17B inhibitors is combined with other treatments dedicated to the same disease. In case the further treatment also corresponds to the administration of a given molecule, said molecule can be present in the same composition as the one containing the PD1 inhibitor and/or the IL-17B inhibitor, or can be administered separately.

In relation to cancer, the other treatment can be e.g.:
- local surgery;
- surgery;
- radiation or radiotherapy;
- chemotherapy;
- immunotherapy;
- targeted therapy, e.g. using BRAF/MEK inhibitors;
- hormone therapy;
- stem cell transplant;
- precision medicine;
- antitumor antibodies;
- gene therapy;
- vaccine;
- cell therapy;
- CAR (chimeric antigen receptor) -T cell therapy;
- TCR (T cell receptor) therapy;
- induction therapy;
- consolidation therapy
- maintenance therapy;
- differentiating agents;
- angiogenesis inhibitors.

Further immunotherapy encompasses other checkpoint inhibitors (CPi) e.g. targeting CTL-4 (e.g. the antibody Ipilimumab (YERVOY^{®})), LAG3, TIM3 and/or TIGIT.

Other immune-oncology (IO) agents include those targeting OX40, GITR, ICOS, VISTA, CD39, CD40, CD47, CD70 (e.g. anti mAbs ARGX-110 and MDX-1203), CD73 or CD137.

Vaccines are also further possible treatments, especially vaccines having PRR (Pattern Recognition Receptors such as Toll-Like Receptors or TLR) agonist properties, e.g. vaccines based on attenuated rotavirus, reovirus or on Newcastle Disease Virus (NDV).

Chemotherapeutic agents to be used with the combination according to the invention include vinca alkaloids, epipodophyllotoxins, anthracycline antibiotics, actinomycin D, plicamycin, puromycin, gramicidin D, paclitaxel (Taxol^{™}, Bristol Myers Squibb), colchicine, cytochalasin B, emetine, maytansine, and amsacrine (or "mAMSA"). The vinca alkaloid class is described in GOODMAN AND GILMAN'S THE PHARMACOLOGICAL BASIS OF THERAPEUTICS (7th ed.), (1985), pp. 1277-1280. Exemplary of vinca alkaloids are vincristine, vinblastine, and vindesine. The epipodophyllotoxin class is described, for example, in GOODMAN AND GILMAN'S THE PHARMACOLOGICAL BASIS OF THERAPEUTICS (7th ed.), (1985), pp. 1280-1281. Exemplary of epipodophyllotoxins are etoposide, etoposide orthoquinone, and teniposide. The anthracycline antibiotic class is described in GOODMAN AND GILMAN'S THE PHARMACOLOGICAL BASIS OF THERAPEUTICS (7th ed.), (1985), pp. 1283-1285. Exemplary of anthracycline antibiotics are daunorubicin, doxorubicin, mitoxantraone, and bisanthrene. Actinomycin D, also called Dactinomycin, is described, for example, in GOODMAN AND GILMAN'S THE PHARMACOLOGICAL BASIS OF THERAPEUTICS (7th ed.), (1985), pp. 1281-1283. Plicamycin, also called mithramycin, is described in Goodmand and Gilman's The Pharmacological Basis of Therapeutics (7th ed), (1985), pp.1287-1288. Additional chemotherapeutic agents include cisplatin (Platinol^{™}, Bristol Myers Squibb), carboplatin (Paraplatin^{™}, Bristol Myers Squibb), mitomycin (Mutamycin^{™}., Bristol Myers Squibb), altretamine (Hexalen^{™}, U.S. Bioscience, Inc.), cyclophosphamide (Cytoxan^{™}, Bristol Myers Squibb), lomustine (CCNU) (CeeNU^{™} Bristol Myers Squibb), carmustine (BCNU) (BiCNU^{™}, Bristol Myers Squibb).

Exemplary chemotherapeutic agents also include aclacinomycin A, aclarubicin, acronine, acronycine, adriamycin, aldesleukin (interleukin-2), altretamine (hexamiethylmelamine), aminoglutethimide, aminoglutethimide (cytadren), aminoimidazole carboxamide, amsacrine (m-AMSA; amsidine), anastrazole (arimidex), ancitabine, anthracyline, anthramycin, asparaginase (elspar), azacitdine, azacitidine (ladakamycin), azaguanine, azaserine, azauridine, 1,1',1"-phosphinothioylidynetris aziridine, azirino(2', 3':3,4)pyrrolo(1,2-a)indole-4,7-dione, BCG (theracys), BCNU, BCNU chloroethyl nitrosoureas, benzamide, 4-(bis(2-chloroethyl)amino)benzenebutanoic acid, bicalutamide, bischloroethyl nitrosourea, bleomycin (blenozane), bromodeoxyuridine, broxuridine, busulfan (myleran), carbamic acid ethyl ester, chlorambucil (leukeran), chloroethyl nitrosoureas, chorozotocin (DCNU), chromomycin A3, cis-retinoic acid, cladribine (2-chlorodeoxyadenosine; 2cda; leustatin), coformycin, cycloleucine, cyclophosphamide anhydrous, chlorambucil, cytarabine, cytarabine, cytarabine HCl (cytosar-u), 2-deoxy-2-(((methylnitrosoamino)carbonyl)amino)-D-glucose, dacarbazine, decarbazine, decarbazine (DTIC-dome), demecolcine, dexamethasone, dianhydrogalactitol, diazooxonorleucine, diethylstilbestrol, docetaxel (taxotere), eflomithine, estramustine, estramustine phosphate sodium (emcyt), ethiodized oil, ethoglucid, ethyl carbamate, ethyl methanesulfonate, fenretinide, floxuridine, floxuridine (fudr), fludarabine (fludara), fluorouracil (5-FU), fluoxymesterone (halotestin), flutamide, flutamide (eulexin), fluxuridine, gallium nitrate (granite), gemcitabine (gemzar), genistein, 2-deoxy-2-(3-methyl-3-nitrosoureido)-D-glucopyranose, goserelin (zoladex), hexestrol, hydroxyurea (hydra), idarubicin (idamycin), ifosfagemcitabine, ifosfamide (iflex), ifosfamide with mesna (MAID), interferon, interferon alfa, interferon alfa-2a, alfa-2b, alfa-n3, interleukin-2, iobenguane, iobenguane iobenguane, irinotecan (camptosar), isotretinoin (accutane), ketoconazole, 4-(bis(2-chloroethyl)amino)-L-phenylalanine, L-serine diazoacetate, lentinan, leucovorin, leuprolide acetate (LHRH-analog), levamisole (ergamisol), mannomustine, maytansine, mechlorethamine, mechlorethamine HCl (nitrogen mustard), medroxyprogesterone acetate (provera, depo provera), megestrol acetate (menace), melengestrol acetate, melphalan (alkeran), menogaril, mercaptopurin, mercaptopurine (purinethol), mercaptopurine anhydrous, MESNA, mesna (mesne), methanesulfonic acid, ethyl ester, methotrexate (mtx; methotrexate), methyl-ccnu, mimosine, misonidazole, mithramycin, mitoantrone, mitobronitol, mitoguazone, mitolactol, mitomycin (mutamycin), mitomycin C, mitotane (o,p'-DDD; lysodren), mitoxantrone HCl (novantrone), mopidamol, N,N-bis(2-chloroethyl)tetrahydro-2H-1,3,2-oxazaphosphorin-2-amine-2-oxide, N-(1-methylethyl)-4-((2-methylhydrazino)methyl)benzamide, N-methyl-bis(2-chloroethyl) amine, nicardipine, nilutamide (nilandron), nimustine, nitracrine, nitrogen mustard, nocodazole, nogalamycin, octreotide (sandostatin), pactamycin, pegaspargase (PEGx-1), pentostatin (2'-deoxycoformycin), peplomycin, peptichemio, photophoresis, picibanil, pipobroman, podofilox, podophyllotoxin, porfiromycin, prednisone, procarbazine, procarbazine HCl (matulane), prospidium, puromycin aminonucleoside, PUVA (psoralen+ultraviolet a), pyran copolymer, rapamycin, s-azacytidine, 2,4,6-tris(1-aziridinyl)-s-triazine, semustine, showdomycin, sirolimus, streptozocin (zanosar), suramin, tamoxifen citrate (nolvadex), taxon, tegafur, tenuazonic acid, TEPA, testolactone, thio-tepa, thioguanine, thiotepa (thioplex), tilorone, topotecan, tretinoin (vesanoid), triaziquone, trichodermin, triethylene glycol diglycidyl ether, triethylenemelamine, triethylenephosphoramide, triethylenethiophosphoramide, trimetrexate (neutrexin), tris(1-aziridinyl)phosphine oxide, tris(1-aziridinyl)phosphine sulfide, tris(aziridinyl)-p-benzoquinone, tris(aziridinyl)phosphine sulfide, uracil mustard, vidarabine, vidarabine phosphate, vinorelbine, vinorelbine tartrate (navelbine), (1)-mimosine, 1-(2-chloroethyl)-3-(4-methylcyclohexyl)-1-nitrosourea, (8S-cis)-10-((3-amino-2,3,6-trideoxy-alpha-L-lyxo-hexopyranosyl)oxy)-7,8,9 ,10-tetrahydro-6,8, 11-trihydroxy-8-(hydroxyacetyl)-1-methoxy-5,12-naphthacenedione, 131-meta-iodobenzyl guanidine (I-131 MIBG), 5-(3,3-dimethyl-1-triazenyl)-1H-imidazole-4-carboxamide, 5-(bis(2-chloroethyl)amino)-2,4(1H,3H)-pyrimidinedione, 2,4,6-tris(1-aziridinyl)-s-thiazine, 2,3,5-tris(1-aziridinyl)-2,5-cyclohexadiene-1,4-dione, 2-chloro-N-(2-chloroethyl)-N-methyl ethanamine, N,N-bis(2-chloroethyl)tetrahydro-2H-1,3,2-oxazaphosphorin-2-amine-2-oxide, 3-deazauridine, 3-iodobenzylguanidine, 5,12-naphthacenedione, 5-azacytidine, 5-fluorouracil, (1aS,8S,8aR,8bS)-6-amino-8-(((aminocarbonyl)oxy)methyl)-1,1a,2,8,8a,8b-hexahydro-8a-methooxy-5-methylazirino(2',3':3,4)pyrrolo(1,2-a)indole-4,7-dione, 6-azauridine, 6-mercaptopurine, 8-azaguanine, and combinations thereof.

Preferred chemotherapeutic agents include e.g. doxorubicin, pemetrexed, a platinium-based drug such as oxaliplatin, cisplatin or carboplatin, paclitaxel, tamoxifen, vincristine, and vinblastine.

Cytokine therapy can also be used, e.g. anti-VEGF mAbs such as bevacizumab, anti-TNFα, anti-IL-6 or anti-TGF-β. Antibodies directed to other isoforms of IL-17, e.g. IL-17A as taught in WO2014/001368, or to EGFR/HER as taught in WO2017/194554 are also of interest.

Infectious diseases are usually treated with antiviral or antimicrobial (e.g. antibiotics) agents. As an example and merely to illustrate the spirit of the invention, the treatment of fungal sepsis, e.g. mucormycosis, can be handled by further administering an antifungal agent such as posaconazole and/or amphotericin, and possibly an immunoadjuvant such as interferon-y.

As further examples, drugs which can be used together with the combination of the invention, especially for treating tuberculosis, are: isoniazid (INH), rifampin/Rifampicin (RIF), ethambutol (EMB) and pyrazinamide (PZA), alone or in association.

As further examples, drugs which can be used together with the combination of the invention, especially for treating HIV infections, are antiretroviral therapies including:
- Nucleoside Reverse Transcriptase Inhibitors (NRTIs) such as abacavir (abacavir sulfate), emtricitabine (FTC), lamivudine (3TC), tenofovir disoproxil fumarate (TDF), zidovudine (azidothymidine, AZT or ZDV);
- Non-Nucleoside Reverse Transcriptase Inhibitors (NNRTIs) such as doravirine (DOR), efavirenz (EFV), etravirine (ETR), nevirapine (NVP), rilpivirine (rilpivirine hydrochloride or RPV);
- Protease Inhibitors (PIs) such as atazanavir (atazanavir sulfate or ATV), darunavir (darunavir ethanolate or DRV), fosamprenavir (fosamprenavircalcoium or DRV), ritonavir (RTV), saquinavir (saquinavir mesylate or SQV), tipranavir (TPV);
- Fusion Inhibitors such as enfuvirtide (T-20);
- CCR5 Antagonists such as maraviroc (MVC);
- Integrase Inhibitors such as dolutegravir (dolutegravirsodium or DTG), raltegravir (raltegravir potassium or RAL);
- Post-Attachment Inhibitors such as ibalizumab;
- Pharmacokinetic enhancers such as cobicistat (COBI).

Such medicine can be used in combination, e.g. as follows:
- abacavir and lamivudine;
- abacavir, dolutegravir and lamivudine;
- abacavir, lamivudine and zidovudine;
- atazanavir and cobicistat;
- bictegravir, emtricitabine and tenofovir alafenamide fumarate;
- darunavir and cobicistat;
- darunavir, cobicistat, emtricitabine and tenofovir alafenamide fumarate ;
- dolutegravir and rilpivirine;
- doravirine, lamivudine and tenofovir disoproxil fumarate;
- efavirenz, emtricitabine and tenofovir disoproxil fumarate;
- efavirenz, lamivudine and tenofovir disoproxil fumarate;
- elvitegravir, cobicistat, emtricitabine and tenofovir alafenamide ;
- elvitegravir, cobicistat, emtricitabine and tenofovir disoproxil fumarate ;
- emtricitabine, rilpivirine and tenofovir alafenamide ;
- emtricitabine, rilpivirine and tenofovir disoproxil fumarate ;
- emtricitabine and tenofovir alafenamide ;
- emtricitabine and tenofovir disoproxil fumarate ;
- lamivudine and tenofovir disoproxil fumarate ;
- lamivudine and zidovudine;
- lopinavir and ritonavir.

As further examples, drugs which can be used together with the combination of the invention, especially for treating HBV/HCV infections, are:
- for HBV: entecavir, lamivudine (3TC), adefovir dipivoxil, interferon alpha-2b, pegylated interferon, telbivudine, tenofovir alafenamide, tenofovir;
- for HCV: ribavirin, daclatasvir, sofosbuvir and velpatasvir, ledipasvir and velpatasvir, telaprevir, interferon alphacon-1, interferon alpha-2b, glecaprevir and pibrentasvir, simeprevir, pegylated interferon, pegylated interferon alpha-2b, interferon alpha-2a, sofosbuvir, ombitasvir and paritaprevir and ritonavir, boceprevir, ombitasvir and paritaprevir and ritonavir and dasabuvir, elbasvir and grazoprevir.

### REFERENCES

Throughout this application, various references describe the state of the art to which this invention pertains. The disclosures of these references are hereby incorporated by reference into the present disclosure.

The invention will be further illustrated by the following figures and examples. However, these examples and figures should not be interpreted in any way as limiting the scope of the present invention.

### FIGURES

**Figure 1****. *In vivo* study of an anti-PD1 therapy in IL-17B WT vs KO mice in the MCA205 fibrosarcoma model**
   Tumor growth (top) and percent survival (bottom) in WT and KO mice treated with control antibodies (IC; A and C) or anti-PD1 antibodies (α-PD1; B and D)
**Figure 2****. *In vivo* study of anti-PD1 and oxaliplatin therapies in IL-17B WT vs KO mice in the MCA205 fibrosarcoma model**
   Tumor growth (top) and percent survival (bottom) in WT and KO mice treated with oxaliplatin (OXA) alone (A and C) or in combination with anti-PD1 antibodies (α-PD1; B and D)
**Figure *3******. In vivo* study of anti-PD1 and anti-IL-17B therapies in the MCA205 fibrosarcoma murine model**
   Tumor growth (top; A and B) and percent survival (bottom; C) in mice treated with:
   - anti-PD1 antibodies (α-PD1) or corresponding control antibodies (2A3); and
   - anti-IL-17B antibodies (α-IL-17B) or corresponding control antibodies (IC).
**Figure 4****. *In vivo* study of an anti-PD1 therapy in IL-17B WT vs KO mice in** the **B16K1 melanoma model**
   Tumor growth (top) and percent survival (bottom) in WT and KO mice treated with control antibodies (IC; A and C) or anti-PD1 antibodies (α-PD1_D6; B and C).

### EXAMPLES

### EXAMPLE 1/ Efficacy of an anti-PD1 immunotherapy in the MCA205 fibrosarcoma model in the presence (WT mice) or absence (IL-17B KO mice) of IL-17B:

### 1-1 Materials and Methods

6-8-week-old C57BL6 WT mice (8) and 8-11-week-old C57BL6 IL-17B KO mice (7) were subcutaneously grafted with 10⁶ MCA205 cells. Tumor growth was monitored using a caliper. At day 6, mice were treated with anti-PD1 antibody (i.p. injections, 200 µg/mouse, BioXCell, RMP1-14, BP0146, batch: 695318A1) or control monoclonal rat IgG2a antibody (i.p. injections, 200 µg/mouse, BioXCell, 2A3, batch: 686318F1B) and then twice a week at day 9, 12, 16, 19, 23, 26 and 30. Animals were sacrificed when tumors reached 1500 mm³ or in case of ulceration.

### 1-2 Results and Conclusion

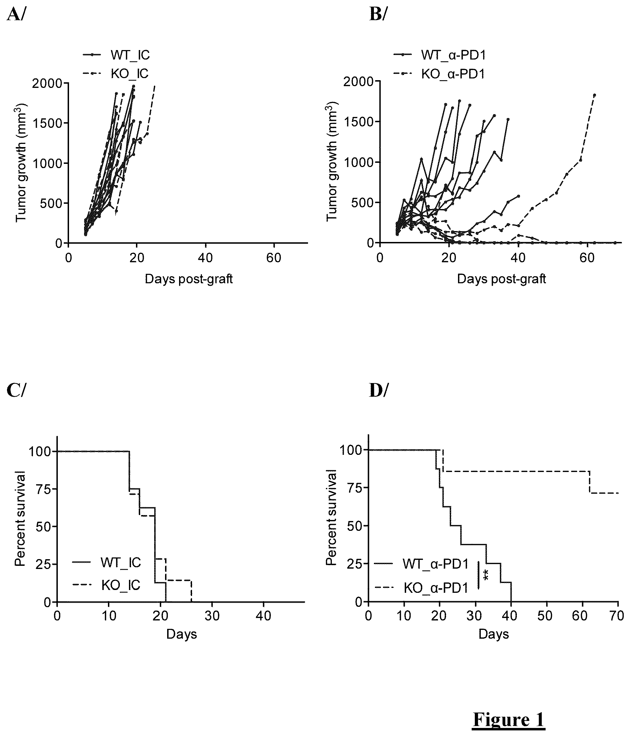

Figure 1 shows the results obtained in terms of tumor growth (top) and percent survival (bottom).

A significant tumor growth delay in IL-17B KO mice treated with anti-PD1 antibody monotherapy was observed compared to WT mice (Fig. 1B). Complete tumor regression was observed in the IL-17B KO background only.

After anti-PD1 treatment, survival of KO mice was significantly increased compared to WT group (Fig. 1D).

In this experiment, whereas anti-PD1 therapy had almost no effect in WT animals, it induced partial and complete responses in the IL-17B KO animals. It therefore confirms the higher efficacy of anti-PD1 monotherapy treatment in IL-17B KO background, i.e. in the absence of IL-17B.

### EXAMPLE 2/ Efficacy of anti-PD1 and oxaliplatin therapies in the MCA205 fibrosarcoma model in the presence (WT mice) or absence (IL-17B KO mice) of IL-17B:

### 2-1 Materials and Methods

6-8-week-old C57BL6 WT mice (8) and 8-11-week-old C57BL6 IL-17B KO mice (7) were subcutaneously grafted with 10⁶ MCA205 cells. Tumor growth was monitored using a caliper. At day 5, mice were treated with oxaliplatin (i.p. injections, 10mg/kg, Accord PX00494, exp. 01/2020) or PBS for control group. The following day (D6), mice were treated with anti-PD1 antibody (i.p. injections, 200 µg/mouse, BioXCell, RMP1-14, BP0146, batch: 695318A1) or control monoclonal rat IgG2a antibody (i.p. injections, 200 µg/mouse, BioXCell, 2A3, batch: 686318F1B) and then twice a week at day 9, 12, 16, 19, 23, 26 and 30. Animals were sacrificed when tumors reached 1500 mm³ or in case of ulceration.

### 2-2 Results and Conclusion

As previously observed in Figure 1A, when animals were grafted with a large number (10⁶) of MCA205 fibrosarcoma cells, no difference was observed in the tumor growth in IL-17B KO compared to WT mice.

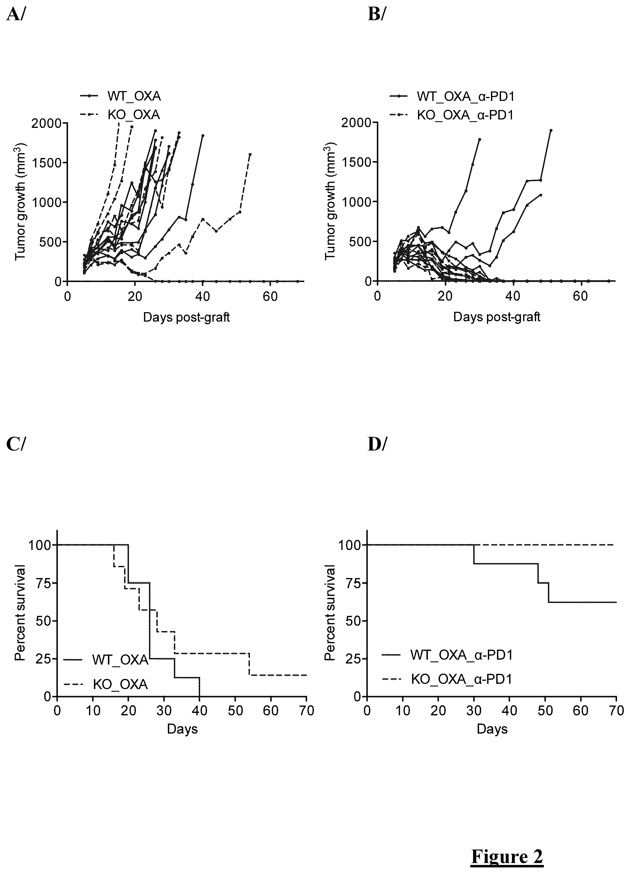

Oxaliplatin treatment alone induced a transient response in WT and IL-17B KO mice. As shown in Figure 2A, its antitumor effect was more pronounced in the IL-17B KO background with 1 out of 7 complete tumor regression.

As previously shown in Figure 1B, the MCA205 fibrosarcoma model is largely resistant to anti-PD1 therapy. Although anti-PD1 treatment slightly delayed tumor progression in the WT mice, it did not induce any complete tumor regression (0%). This is in sharp contrast with the results obtained in the IL-17B KO background in which the anti-PD1 therapy induced complete tumor regression in 4 out of 7 (57%) IL-17B KO mice at the end of the study and 1 almost complete regression of another tumor which never regrew. At the end of the study, the survival of the IL-17B KO mice treated with anti-PD1 antibody was significantly improved (72%) compare to WT mice (0%) treated with anti-PD1 antibody (Fig. 1D).

As revealed by Figure 2B, the combination of oxaliplatin and anti-PD-1 was more effective and induced complete tumor regressions in all IL-17B KO mice (7 out of 7, 100%) compared to 5 out of 8 (63%) WT mice.

In the PD-1 resistant fibrosarcoma model, the high efficacy of anti-PD1 monotherapy treatment was demonstrated in IL-17B KO background only (Fig. 1D). Furthermore and as revealed by

Figure 2D, combination with oxaliplatin was able to induce 100% of survival in the IL-17B KO animals whereas it exhibited some efficacy in WT mice (63% of survival).

This example clearly reveals the benefit to combine the approach according to the invention (inhibition of PD1 and IL-17B) with further therapeutic approach, e.g. chemotherapy.

### EXAMPLE 3/ Efficacy of anti-PD1 and anti-IL-17B immunotherapies in the MCA205 fibrosarcoma model :

In order to confirm the results obtained in example 1 wherein the absence of IL-17B was obtained by the use of an IL-17B KO mouse, the experiment was repeated in WT mice treated with anti-IL-17B antibodies.

### 3-1 Materials and Methods

8-week-old C57BL6 mice were subcutaneously grafted with 5.10⁴ MCA205 cells, 10 animals per group (9 animals for IC groups). Tumor growth was monitored using a caliper. At day 5, mice were treated with anti-IL-17B antibody (s.c. injections, 200 µg/mouse) or control antibody (s.c.. injections, 200 µg/mouse) +/- anti-PD1 antibody (i.p. injections, 200 µg/mouse, BioXCell, RMP1-14, BP0146, batch: 640517M2B) or control monoclonal rat IgG2a antibody (i.p. injections, 200 µg/mouse, BioXCell, 2A3, batch: 627416N1) and then twice a week at day 9, 12, 16, 19, 23, 26, 30, 33, 37, 40, 44, 47, 51, 54, 58, 62, 65, 68 and 72 for anti-IL-17B/IC antibodies and twice a week at day 9, 12 and 16 for anti-PD1/IC antibodies. Animals were sacrificed when tumors reached 1500 mm³ or in case of ulceration.

### 3-2 Results and Conclusion

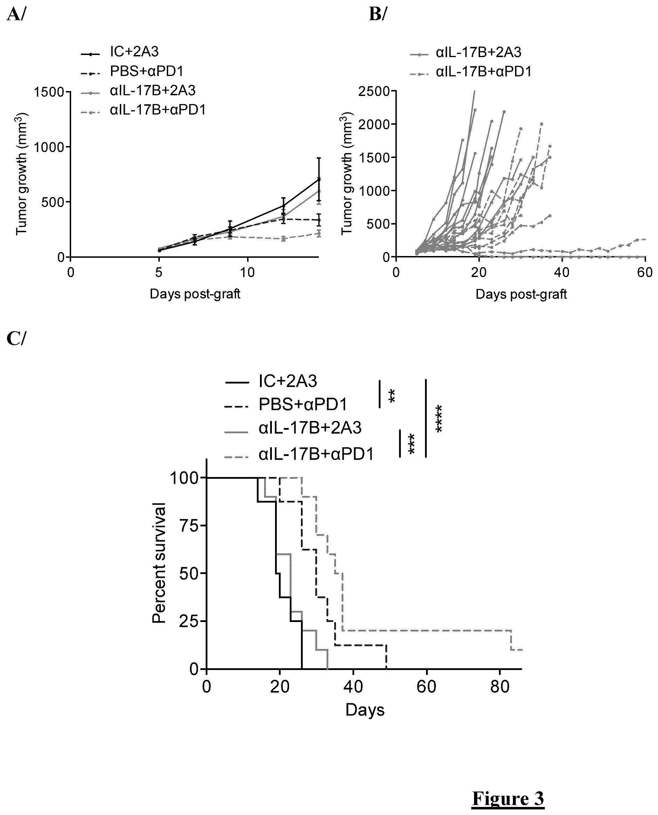

As shown previously, the MCA205 model is largely resistant to anti-PD1 therapy. Accordingly, anti-PD1 treatment alone had a modest but significant impact on tumor growth (Fig. 3A) and did not affect survival (0% at the end of the study Fig. 3C). In this experiment, the curative treatment with an anti-IL-17B antibody did not significantly affect MCA205 tumor growth (Fig. 3B). However, the anti-IL-17B antibody significantly increased the response to anti-PD1 therapy including 1 out of 10 (10%) complete regression and 1 additional long-term control of a near-complete response (10%) (Fig. 3B).

In line with the data obtained using the IL-17B KO mice, anti-IL-17B antibody increased the response to anti-PD1 therapy and induced long term responses (complete and long-term near complete responses). Combination of anti-IL-17B antibody and anti-PD-1 antibody significantly improved mouse survival, whereas anti-PD1 therapy alone did not (Fig. 3C).

### EXAMPLE 4/ Efficacy of an anti-PD1 immunotherapy in the B16K1 melanoma model in the presence (WT mice) or absence (IL-17B KO mice) of IL-17B:

In order to confirm the results obtained in example 1 in a fibrosarcoma model, the experiment was repeated in a melanoma model.

### 4-1 Materials and Methods

10-week-old C57BL6 WT mice (10) and 10-week-old C57BL6 IL-17B KO mice (7 or 8) were subcutaneously grafted with 10⁵ B16K1 melanoma cells. Tumor growth was monitored using a caliper. At day 6, mice were treated with anti-PD1 antibody (i.p. injections, 200 µg/mouse, BioXCell, RMP1-14, BP0146, batch: 695318A1) or control monoclonal rat IgG2a antibody (i.p. injections, 200 µg/mouse, BioXCell, 2A3, batch: 686318F1B) and then twice a week at day 6, 10 and 13. Animals were sacrificed when tumors reached 1500 mm³ or in case of ulceration.

### 4-2 Results and Conclusion

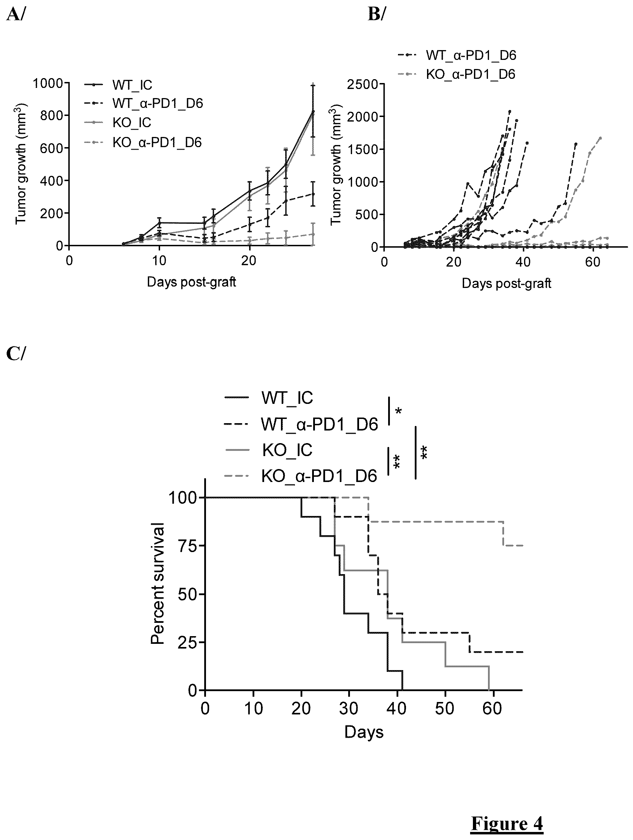

As previously observed, the data shown on Figure 4 reveal that the effect of the anti PD1 antibody is improved in the KO background.

This example validates that the PD1/ IL-17B inhibitory approach according to the invention can be applied to other types of cancer or even to other diseases.

## Claims

1. A composition comprising an inhibitor of PD1 or of a ligand thereof and an inhibitor of IL-17B or of a receptor thereof.

2. A composition according to claim 1 wherein the inhibitor of PD1 or of a ligand thereof is present in an amount inferior to its amount in a composition not comprising an inhibitor of IL-17B or of a receptor thereof.

3. A composition according to claim 1 or 2 for use in therapy, advantageously in treating a cancer or an infectious disease.

4. A composition comprising an inhibitor of IL-17B or of a receptor thereof for use in treating a cancer or an infectious disease in a subject treated with an inhibitor of PD1 or of a ligand thereof.

5. A composition for its use according to claim 4 wherein the treatment is for increasing the sensitivity of the subject to the inhibitor of PD1 or of a ligand thereof.

6. A composition for its use according to claim 4 or 5 wherein the subject is resistant to the treatment with the inhibitor of PD1 or of a ligand thereof.

7. A composition for its use according to any of claims 3 to 6, wherein the infectious disease is selected in the group consisting of: severe sepsis, septic shock, viral infections especially infections due to human immunodeficiency virus, hepatitis virus, cytomegalovirus or Epstein-Barr virus, fungal infections such as mucormycosis, mosquito-borne infectious diseases such as malaria, and bacterial infections such as tuberculosis.

8. The composition for its use according to claim 7 wherein the infectious disease is selected in the group consisting of: tuberculosis, malaria, HIV infection, HBV infection and HCV infection.

9. The composition for its use according to any of claims 3 to 6 wherein the cancer is resistant to inhibitors of PD1 or of a ligand thereof.

10. The composition for its use according to any of claim 3 to 6 and 9 wherein the cancer is selected from the group consisting of: melanoma, sarcoma, Cutaneous Squamous Cell Carcinoma (CSCC), Primary Mediastinal Large B-Cell Lymphoma (PMBCL), Microsatellite Instability-High Cancer (MSI-H), Hepatocellular Carcinoma (HCC), Small Cell Lung Cancer, Non-Small Cell Lung Cancer (NSCLC), ovarian cancer, Merkel Cell Carcinoma (MCC), Head and Neck Squamous Cell Carcinoma (HNSCC), Cervical Cancer, gastric cancer, esophageal cancer, urothelial carcinoma, Renal Cell Carcinoma (RCC), bladder carcinoma, anal cancer, triple negative breast cancer (TNBC), mesothelioma and Hodgkin's lymphoma (cHL).

11. A composition according to claim 1 or 2 or a composition for its use according to any of claims 3 to 10, wherein the inhibitor of PD1 or of a ligand thereof is an inhibitor of PD1 or of PDL1 or of PDL2, advantageously an anti-PD1 antibody or an anti-PDL1 antibody.

12. A composition according to claim 11 or a composition for its use according to claim 11, wherein the inhibitor of PD1 or of a ligand thereof is selected from the group consisting of Pembrolizumab, Nivolumab, BMS-936559, Cemiplimab, Avélumab, Durvalumab, Atezolizumab, Spartalizumab, or their combination.

13. A composition according to claim 1, 2, 11 or 12 or a composition for its use according to any of claims 3 to 12 wherein the inhibitor of IL-17B or of a receptor thereof is an antibody directed against IL-17B.

14. A composition according to claim 1 or 2 or a composition for its use according to any of claims 3 to 10 wherein the inhibitor is an inhibitor of the expression of IL-17B or of a receptor thereof or of PD1 or of a ligand thereof, advantageously a siRNA or an antisense oligonucleotide.

15. A composition as defined in any of the preceding claims as a combined preparation for simultaneous, separate or sequential use in therapy, advantageously in treating a cancer or an infectious disease.
